(19) Europäisches Patentamt
European Patent Office
Office européen des brevets

(11) **EP 4 574 801 A1**

(12) **EUROPEAN PATENT APPLICATION**

(43) Date of publication:
**25.06.2025 Bulletin 2025/26**

(21) Application number: **23220106.1**

(22) Date of filing: **22.12.2023**

(51) International Patent Classification (IPC):
**C04B 40/00** [(2006.01)] **G06F 30/20** [(2020.01)]
**G16C 20/30** [(2019.01)] **G16C 60/00** [(2019.01)]
**G06F 111/10** [(2020.01)]

(52) Cooperative Patent Classification (CPC):
**G16C 60/00; C04B 40/0032; G06F 30/20;**
**G06Q 50/08;** G06F 2111/10; G16C 20/30

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Sika Technology AG**
**6340 Baar (CH)**

(72) Inventors:
• **VELTEN, Ulf**
  **8955 Oetwil an der Limmat (CH)**
• **WEINKAUF, Annette**
  **5603 Staufen (CH)**
• **ZIMMERMANN, Jörg**
  **8400 Winterthur (CH)**

(74) Representative: **Sika Patent Attorneys**
**C/o Sika Technology AG**
**Corp. IP Dept.**
**Tüffenwies 16**
**8048 Zürich (CH)**

(54) **SYSTEMS AND METHODS TO DETERMINE CONSTRUCTION COMPOSITIONS AND THE ASSOCIATED ENVIRONMENTAL IMPACT**

(57) The present invention relates to computer implemented methods and systems comprising a computer device to determine a construction composition and the associated environmental impact. The present invention also relates to computer implemented methods and systems comprising a computer device to determine a construction admixture suitable to produce construction compositions according to specification and to provide the environmental impact associated with the cured construction composition.

Figure 1

**Description**

**Technical Field**

The present invention relates to computer implemented methods to determine a construction composition and the associated environmental impact.

**Background**

In the construction industry, curable construction compositions are widely used for various applications. Examples of such compositions are mortars, concrete, grout, or screed compositions.

It is common practice to determine mix designs for construction compositions suitable to meet certain specifications such as performance requirements of the construction composition. Typically, a dry mix comprising a mineral binder and preferably additionally comprising aggregates and/or fillers is determined, and one or more admixtures are added to meet these performance requirements.

In recent years, computer implemented methods have been developed to manage and to determine mix designs for construction compositions according to specifications. Such methods are for example disclosed in US 2020/0402619 A1 (Verifi LLC) or in WO 2020/167728 A1 (Construction Research & Technology GmbH).

However, known methods to determine construction compositions according to specification do not take into account the different environmental impact that such construction composition may have. However, the environmental footprint, especially over the whole life cycle of a construction composition, has become a very important aspect and must be considered by specifiers and producers of construction compositions.

It is therefore desirable to develop methods, in particular computer implemented methods, that are able to determine construction compositions according to specification and that take into account the environmental impact thereof.

**Summary of the invention**

It is an object of the present invention to provide computer implemented methods to determine a construction composition according to specification, i.e. that meets certain performance requirements. In particular, the construction composition comprises a construction mixture, which may be set according to local availabilities and needs, and an admixture which may be variable. The computer implemented method of the present invention should provide an environmental impact associated with the cured construction composition and preferably provide for the optimization of the construction composition to lower environmental impact.

It is another object of the present invention to provide computer implemented methods to determine a construction admixture suitable for a construction composition and to provide the environmental impact associated with the cured construction composition.

It is also an object of the present invention to provide systems for the production of construction compositions and/or admixtures determined by computer implemented methods of the invention.

The object of the present invention is solved by a method as claimed in claim 1.

In particular, the methods of the present invention comprise steps of simulating and outputting for each admixture an environmental impact of the cured construction composition comprising the construction mixture and the respective admixture.

Further aspects of the present invention are the subject matter of independent claims.

Preferred embodiments are the subject matter of dependent claims.

**Detailed Ways**

In a first aspect the present invention relates to a computer implemented method to determine a construction composition which comprises a construction mixture and an admixture, said method comprising the steps of

  a) receiving a specification for the construction composition, said specification comprising at least one performance requirement for the construction composition,
  b) accessing at least one input parameter related to the construction mixture,
  c) providing the specification for the construction composition and at least one input parameter related to the construction mixture to a predictive model,
  d) determining, using the predictive model, a plurality of admixtures suitable to be added to the construction mixture to meet the performance requirement of the construction composition,

e) simulating for each admixture an environmental impact of the cured construction composition comprising the construction mixture and the respective admixture,

f) outputting the plurality of construction compositions and the respective environmental impact of the cured construction compositions.

A construction composition within the present context is a material comprising a construction mixture and an admixture and optionally additionally comprising water. It can be preferred that the construction composition consists of a construction mixture and an admixture. Preferably, the construction composition is a mortar, concrete, grout, or screed composition.

A construction mixture within the present context is a material comprising at least one mineral binder and preferably additionally comprising aggregates and/or fillers. A mineral binder can cure by a chemical reaction after addition of mixing water to form a solid. In particular a mineral binder reacts in the presence of water in a hydration reaction to form solid hydrates or hydrate phases. This can be, for example, a hydraulic binder, for example cement or hydraulic lime, a latent hydraulic binder, for example slag, a pozzolanic binder, for example fly ash or clays, or a non-hydraulic binder, for example gypsum or white lime. Cement in particular can be selected from the group consisting of Portland cement, calcium aluminate cement, calcium sulfoaluminate cement, and mixtures thereof.

A construction mixture preferably is in dry form. A construction mixture typically starts to set and cure when contacted with water. However, a construction mixture may also be in a wet form.

Aggregates can be any material that is non-reactive in the hydration reaction of mineral binders. Aggregates can be any aggregate typically used for construction materials. Typical aggregates are for example rock, crushed stone, crushed concrete, gravel, sand, especially quartz sand, river sand and/or manufactured sand, glass, expanded glass, hollow glass beads, glass ceramics, quarry wastes, porcelain, electro fused or sintered abrasives, firing support, silica xerogels, bio sourced aggregates such as hemp or sunflower. An especially preferred aggregate is sand. Aggregates may have a particle size of between 0.063 mm and 63 mm.

Fillers are characterized by a small particle size. For example, fillers are selected from fine calcium carbonate, ground limestone, ground dolomite, and/or ground aluminum oxide, and/or non-reactive slags. Typically, fillers have a particle size of below 0.063 mm.

It is possible to use more than one aggregate and more than one filler also in combination.

According to embodiments, a construction mixture of the present invention comprises at least one cement, at least one aggregate, and at least one filler. For example, Portland cement, sand, and limestone filler.

According to embodiments, admixtures are selected from constituents belonging to one or more of the following groups: plasticizers, superplasticizers, shrinkage reducers, air-pore forming agents, air entrainers, de-aerating agents, stabilizers, viscosity modifiers, thickeners, water reducers, water retention aids, retarders, water resisting agents, hydrophobic agents, fibers, blowing agents, defoamers, redispersible polymer, dedusting agents, shrinkage reducers, chromate reducers, pigments, biocides, corrosion inhibitors, and steel passivating agents. An admixture within the present context in particular is a material according to standard EN 934. It is possible to combine one, two, three, or more constituents into one admixture. Preferably, an admixture of the present invention comprises two or more constituents, for example a combination of superplasticizer and a retarder, or a combination of accelerator and retarder, or a combination of two chemically different superplasticizers.

An admixture can be in the form of a solid or in the form of a dispersion or solution, especially in water.

According to embodiments, the at least one performance requirement for the construction composition is selected from at least one processing requirement, at least one strength requirement, at least one exposure requirement, at least one environmental requirement, at least one cost requirement, and/or at least one real-time sensor reading.

According to embodiments, the processing requirement is selected from rheological properties, especially the desired slump class, compaction class, flow class or slump-flow class, viscosity class, sedimentation class according to DIN EN 206:2013+A2:2021. However, alternatively or in addition, other processing requirements might be considered as well.

According to embodiments, the exposure requirement is selected from an exposure class according to DIN EN 206:2013+A2:2021, especially selected from class XO, XC1, XC2, XC3, XC4, XS1, XS2, XS3, XD1, XD2, XD3, XF1, XF2, XF3, XF4, XA1, XA2, XA3. However, alternatively or in addition, other exposure requirements might be considered as well. The exposure requirement may also be another requirement relating to durability of the construction composition.

According to embodiments, the strength requirement is selected from the compressive strength classes according to DIN EN 206: 2013+A2:2021, especially selected from C8/10, C12/15, C16/20, C20/25, C25/30, C30/37, C35/45, C40/50, C45/55, C50/60, C55/67, C60/75, C70/85, C80/95, C90/105, C100/115, LC 8/9, LC12/13, LC16/18, LC20/22, LC25/28, LC30/33, LC35/38, LC40/44, LC45/50, LC50/55, LC55/60, LC60/66, LC70/77, LC80/88.

According to embodiments, the environmental requirement is selected from the temperature, humidity, solar irradiance, air pressure, wind speed, wind direction, atmospheric composition, altitude, and/or other weather parameters at the place of producing the curable binder composition, at the place during the transport of the curable binder composition, at the place of intermediate storage of the curable binder composition, and/or at the place of application of the curable binder composition. Also, the at least one environmental requirement can reflect the time between production and application the curable binder composition, the time of transport of the curable binder composition, the time of intermediate storage of the curable binder composition, and/or traffic conditions during transport of the curable binder composition, e.g. traffic density and/or average speed of vehicles on a transport route during the transport of the curable binder composition. In particular the at least one environmental requirement at least reflects environmental conditions at the place of application of the curable binder composition, especially at a job site. Even more preferred, the at least one environmental requirement in addition reflects environmental conditions during transport and/or intermediate storage, and further preferred also during manufacturing of the curable binder composition. This ensures that the curable binder composition has the right properties for the specific conditions are the job site.

A performance requirement can, for example, be captured by a sensor. In particular, a performance requirement can be a real-time sensor reading.

In particular, the at least one input parameter is selected from chemical and/or physical properties of at least one constituent of the construction mixture.

According to embodiments, the at least one input parameter related to the construction mixture is selected from a chemical and/or physical property of at least one individual constituent of the construction mixture.

According to embodiments, the at least one input parameter related to the construction mixture is accessed by measurement. Measurement can be done discontinuously or continuously. Measurement can in particular be done by means of physico-chemical analysis, sensors, or imaging, especially in real time.

For example, the at least one input parameter is selected from the type and amount of mineral binder present, the alkaline content of the mineral binder, the type and amount of aggregate and/or filler, and/or a particle shape and/or size of the aggregates and/or fillers.

For example, the amount can be expressed in a weight ratio, a volume ratio, or a molar ratio.

For example, the type of cement can be according to standards DIN EN 197-1:2011, ASTM C150, or CSA A3000-08, in particular selected from types CEM I, CEM II, CEM III, CEM IV, CEM V or CEM VI.

For example, the particle shape can be according to standards EN 933-1:2012 to - 7:2012.

In particular the at least one performance requirement and/or the at least one input parameter is represented by one or more numerical values. Alternatively, the at least one performance requirement and/or at least one input parameter may be captured in the form of a number of strings and/or symbols and later assigned numerical values. This will in particular facilitate the manual capturing of the at least one performance requirement and/or at least one input parameter by users.

Within the present context a predictive model is an algorithm which may be selected from linear regression models, logistic regression models, decision trees, random forests, k-nearest neighbors, k-means clustering, principal component analysis, genetic algorithms, hidden Markov models, XGBoost, Gaussian mixture models, and/or machine learning algorithms or artificial intelligence such as supported vector machines, neural networks, autoencoders.

In particular, the predictive model is trained on a set of data points comprising proportion and type of admixtures and the at least one input parameter as independent variables and measured performance requirements and environmental impact as dependent variables. Thereby, for example, the predictive model generates a number of functional relationships suitable to determine admixture suitable to be added to the construction mixture to meet the performance requirement of the construction composition. In particular, the functional relationships are related to one or more input parameters. In other words, there is a correlation between the functional relationship and the construction mixture.

Once the predictive model is trained, a data set representing a specification for the construction composition may be received. The data set comprises at least one, preferably two or more performance requirements of the construction composition. Performance requirements may optionally be prioritized. Additionally, another dataset comprising at least one input parameter, preferably two or more input parameters, is accessed. Performance requirements and/or input parameters may be provided by a human user via a user interface. Thereby, in particular, the user interface is configured such that for at least one requirement and/or parameter a predefined list of selectable requirements and/or parameters is presented to a user. It is also possible that performance requirements and/or input parameters are provided by a machine interface of a computer system such as a database or the Internet. It is also possible that certain performance requirements and/or input parameters are provided by a human while others are provided by a computer system.

The datasets comprising the at least one performance requirements and at least one input parameter are provided to the predictive model. The predictive model then determines a plurality of admixtures that are suitable to be added to the construction mixture to meet the performance requirement of the construction composition.

For example, the predictive model may use functional relationships generated during training, for example by regression analysis or machine learning approaches, to determine the plurality of admixtures.

According to embodiments the functional relationship for the admixture is selected form a list of different predefined multivariable functions with at least two variables, whereby the selection of the functional relationship is made depending on the at least one input parameter and the performance requirements are used as the variables in the selected multivariable function to calculate the respective admixture concentration.

Determination of an admixture especially means the determination of a list of raw materials making up the admixture, amounts or ratios of the admixture raw materials, any mixing techniques used to mix the raw materials to create the admixture, and a ratio, in particular a weight ratio, of the admixture relative to the construction mixture or relative to the construction composition.

It is possible that the predictive model outputs the admixtures determined via a user interface, via a machine interface and/or on a data storage medium. The output may specify a list of raw materials making up the admixture, amounts or ratios of the admixture raw materials, and any mixing techniques used to mix the raw materials to create the admixture.

The number of admixtures determined is in principle not limited. It may, however, be advantageous to limit the number of admixtures determined to a total number, such as, for example 100 or 100'000 or 1'000'000. Alternatively, it may be advantageous to limit the time available for determination and thereby limiting the number of admixtures to the number that can be performed within the given time.

For each admixture determined, a simulation of the environmental impact of the cured construction composition comprising the construction mixture and the respective admixture is performed. Thereby, a plurality of construction compositions is simulated. This simulation can be performed by the same predictive model used for the determination of the admixture or separately therefrom, for example by a different predictive model.

A cured construction composition is obtained by mixing a construction composition with water and letting it cure until it has obtained its final mechanical strength. Curing can be at ambient conditions or with elevated temperature and/or pressure.

The environmental impact preferably is selected from one or more of the following parameters: global warming potential, carbon footprint, depletion potential of the stratospheric ozone layer, acidification potential of land and water, eutrophication potential, formation potential of tropospheric ozone photochemical oxidants, abiotic depletion potential for non-fossile resources, abiotic depletion potential for fossil resources, use of fresh water resources, use of renewable energy, use of non-renewable energy, hazardous or non-hazardous waste disposal, radioactive waste disposal, release of volatile organic compounds, release of fine dust, use and release of carcinogenic or mutagenic materials, use and release of toxic materials, use and release of persistent materials. The environmental impact of the construction composition especially is an environmental product declaration (EPD). Preferably, an EPD of the present invention is according to standard ISO 14025.

According to preferred embodiments, the environmental impact is selected from the global warming potential or the carbon footprint, in particular in $CO_2$ equivalents in kg per $m^3$. The global warming potential or the carbon footprint may include or exclude biogenic carbon and fossil carbon.

The simulation of the environmental impact may include one or more of the following steps:

- specifying of the functional unit which represents the quantified performance of the product, for example one $m^3$ of a construction composition,

- identification of inputs, for example energy and raw materials, and outputs, for example emissions and waste, for each stage of the life cycle of the construction composition,

- including weighing factors for inputs and outputs,

- assessing material related data, for example environmental impact data for raw materials, from data bases, manufacturers, or the Internet,

- presenting the simulation result in a standardized format according to standard ISO 14025.

The plurality of construction compositions and the respective environmental impact is outputted via a user interface, via a machine interface and/or on a data storage medium. The output may be in the form of a display. The output may specify a list of raw materials making up the respective construction composition or construction mixture and admixture, amounts or ratios of raw materials, and any mixing techniques used to mix the raw materials. The output, in particular the environmental impact, may be represented by one or more numerical values or in the form of a number of strings and/or symbols.

The construction compositions of the plurality of construction compositions may be displayed together with their respective environmental impact. It is possible, that only those characteristics that differ from construction composition to construction composition may be displayed together with the respective environmental impact of the cured construction composition. According to embodiments, the plurality of construction compositions can be sorted and optionally displayed by increasing or decreasing environmental impact.

In addition to the environmental impact, the cost of a construction composition may additionally be simulated and optionally displayed.

A user may select one of the construction compositions for use in a project. In some embodiments, a construction composition may automatically be selected. Selection may be made based on lowest environmental impact. Selection may also be made based on other criteria, for example chemical composition of the construction composition.

A method of the present invention significantly reduces or eliminates the number of experiments that need to be run on a given mixture or set of construction compositions, since the predictive model can eliminate construction compositions that are unlikely to meet the job requirements. Additionally, the use of simulations substitutes for or supplements experiments.

Furthermore, embodiments of the present invention are better able to arrive at a set of optimized construction mixture proportions, since many more variables can be taken into account in the process of designing the construction composition. Also, different input parameters can be weighed against each other so that improved combinations or synergies can be identified. Because cost may be considered as a factor, the resulting solution may be less expensive and less prone to over-engineering.

Also, embodiments of the present invention can rapidly optimize the construction compositions around multiple different performance requirements. The effects on a change in the construction composition can be immediately evaluated across multiple different performance requirements. Again, this reduces significantly the number of experiments that need to be done.

Finally, existing construction compositions can be re-optimized quickly based on changing performance requirements and/or input parameters. This allows for improved quality control and more consistent product as compared to traditional methods.

According to embodiments, a method of the present invention additionally comprises the step of

g) deleting from the plurality of construction compositions at least one which has not the lowest environmental impact.

Preferably, step g) is done prior to step f) of a method of the present invention. That is, preferably step g) is done after the simulation of environmental impact but before outputting in step f). Thereby, it is possible that a limited number of construction compositions is outputted. It is, for example, possible to delete all construction compositions and their respective environmental impact from the plurality simulated with exception of the one with the lowest environmental impact. In other words, the plurality of construction compositions can be limited to only one. It is, of course, also possible to delete all construction compositions not having the lowest environmental impact and thereby limiting to a number of different construction compositions all having the same environmental impact.

According to embodiments, a method of the present invention additionally comprises the steps of

h) changing at least one input parameter related to the construction mixture in step b),
i) repeating steps c) to f) and optionally g),
j) comparing the lowest environmental impact of the cured construction compositions outputted in step f) and in step i),
k) repeating steps h) to j) until the comparison in step j) shows no further reduction in environmental impact of the cured construction compositions,
l) outputting the construction compositions and the respective environmental impact of the cured construction compositions obtained in step k).

In the alternative it is possible that in step k) the steps h) to j) are repeated for a predetermined number of iterations. For example 100 or 100'000 or 1'000'000 iterations. In the alternative it is possible that in step k) the steps h) to j) are repeated for a predetermined period of time. Thereby limiting the number of iterations to the number of repeats that can be performed within the given time. In the alternative it is possible that in step k) the steps h) to j) are repeated until a predetermined threshold of the environmental impact is met.

It is possible to change the at least one input parameter in a targeted way. Especially, the at least one input parameter can be changed in a way that is likely to result in a lower environmental impact. One example would be to change a constituent of the construction mixture to another constituent which is known to have a lower environmental impact. Another example would be to lower the amount of a constituent of the construction mixture which is known to have a high environmental impact.

It is in particular possible in step h) to change the type and amount of mineral binder present, the alkaline content of the mineral binder, the type and amount of aggregate and/or filler, and/or a particle shape and/or size of the aggregates and/or fillers.

According to embodiments, the method can be implemented in various ways, for example in the form of a standalone application without need for any further resources such as for example server systems. This is in particular helpful in areas with limited access to communication networks. However, the method can be implemented as well in a distributed computing environment, for example as a client in combination with a dedicated server as a storage unit and/or with a specialized processing unit.

Also, the inventive method can be implemented in a flexible manner with known software architectures, for example as native application, a progressive web application (PWA), or a hybrid application (combination of native and PWA). Also, the inventive method can be implemented in one single application, or it may be divided into two or more separate applications with appropriate software interfaces for data exchange between the applications. Furthermore, the application(s) can be extended with additional functions in a flexible manner.

Applications can be implemented for any kind of operating systems such as e.g. iOS, Android, Microsoft Windows and/or Linux.

In another aspect the present invention relates to a non-transitory computer-readable medium storing instructions that, when executed by one or more processors, cause the one or more processors to

i) receive a specification for a construction composition comprising a cementitious mixture and an admixture, said specification comprising at least one performance requirement for the construction composition,
ii) access at least one input parameter related to the construction mixture,
iii) provide the specification for the construction composition and at least one input parameter related to the construction mixture to a predictive model,
iv) determine, using the predictive model, a plurality of admixtures suitable to be added to the construction mixture to meet the performance requirement of the construction composition,
v) simulate for each admixture an environmental impact of the cured construction composition comprising the construction mixture and the respective admixture,
vi) output the plurality of construction compositions and the respective environmental impact of the cured construction compositions.

All features and embodiments described above also apply to this aspect.

Examples of a computer-readable storage medium may include any tangible media capable of storing electronic data, including volatile memory or non-volatile memory, removable or non-removable memory, erasable or non-erasable memory, writeable or rewriteable memory, and so forth. Embodiments may also be at least partly implemented as instructions contained in or on a non-transitory computer-readable medium, which may be read and executed by one or more processors to enable performance of the operations described herein.

According to embodiments, the non-transitory computer-readable medium may be selected from hard disk drives, solid state drives, CD-ROM, DVD, Blu-ray discs, USB Flash Drives, memory cards, magnetic tapes, floppy disks, EPROM, optical drives, magnetic diskettes, compact flash cards, secure digital cards, external hard drives, network attached storage, cloud storage, RAID, 3D XPoint memory.

The invention further includes any of the described mediums herein, further storing instructions for: receiving a notification that the input parameter is changed and reoptimizing the determined construction composition based on the notification. The invention further includes any of the described mediums herein, wherein the changed input comprises a different set of available raw materials and components than were available when the construction composition was first determined.

The invention further includes an apparatus comprising a non-transitory computer-readable storage medium storing logic for a predictive model configured to select a combination of construction mixtures and admixtures, a hardware interface configured to receive training data, the training data comprising a predefined construction composition and associated performance characteristics for the predefined construction composition, and a hardware processor circuit configured to train the predictive model based on the training data, receive, via the interface, one or more performance requirements for a new construction composition, and use the predictive model to select the new construction composition based on the received performance requirements, wherein the new construction composition is output using the interface.

In another aspect, the present invention relates to a computer implemented method to determine a construction admixture, said method comprising the steps of

1) receiving a specification for a construction composition comprising a construction mixture and the construction admixture, said specification comprising at least one performance requirement for the construction composition,
2) accessing at least one input parameter related to the construction admixture,
3) accessing at least one input parameter related to the construction mixture,
4) providing the specification for the construction composition and the at least one input parameter related to the construction admixture and the construction mixture to a predictive model,
5) determining, using the predictive model, a plurality of admixtures suitable to be added to the construction mixture to meet the performance requirement of the construction composition,
6) simulating for each admixture an environmental impact of the cured construction composition comprising the construction mixture and the respective admixture,
7) outputting the plurality of construction admixtures and the respective environmental impact of the cured construction compositions.

All features and embodiments described above also apply to this aspect.

The terms "admixture" and "construction admixture" may be used interchangeably within the present context.

A construction admixture within the present context in particular is a material according to standard EN 934. It is possible to combine one, two, three, or more constituents into one admixture.

Preferably, the at least one input parameter is selected from chemical and/or physical properties of at least one constituent of the construction admixture.

According to embodiments, the at least one input parameter related to the construction admixture is accessed by measurement. Measurement can be done discontinuously or continuously. Measurement can in particular be done by means of physico-chemical analysis, sensors, or imaging, especially in real time.

For example, the at least one input parameter is selected from the type and amount of at least one constituent of the admixture.

For example, the amount can be expressed in a weight ratio, a volume ratio, or a molar ratio.

It is possible that the method to determine a construction admixture further comprises a step of

8) selecting one or more characteristics in which the plurality of construction admixtures differs, the one or more characteristics not being an input parameter of the construction admixture and outputting a comparison of the plurality of construction admixtures based on the selected characteristics.

The one or more characteristics can be selected from the group consisting of cost, material availability, environmental impact, shelf life, physical state such as solid or liquid, and packaging required.

Outputting may include sorting of the construction admixture according to environmental impact of the cured construction composition and/or at least one selected characteristic.

In another aspect, the present invention relates to a non-transitory computer-readable medium storing instructions that, when executed by one or more processors, cause the one or more processors to

1) receive a specification for a construction composition comprising a construction mixture and a construction admixture, said specification comprising at least one performance requirement for the construction composition,
2) access at least one input parameter related to the construction admixture,
3) access at least one input parameter related to the construction mixture,
4) provide the specification for the construction composition and the at least one input parameter related to the construction admixture and the construction mixture to a predictive model,
5) determine, using the predictive model, a plurality of construction admixtures suitable to be added to the construction mixture to meet the performance requirement of the construction composition,
6) simulate for each admixture an environmental impact of the cured construction composition comprising the construction mixture and the respective construction admixture,
7) output the plurality of construction admixtures and the respective environmental impact of the cured construction compositions.

All features and embodiments described above also apply to this aspect.

In particular, the non-transitory computer-readable medium may be selected from hard disk drives, solid state drives, CD-ROM, DVD, Blu-ray discs, USB Flash Drives, memory cards, magnetic tapes, floppy disks, EPROM, optical drives, magnetic diskettes, compact flash cards, secure digital cards, external hard drives, network attached storage, cloud storage, RAID, 3D XPoint memory.

According to embodiments, a non-transitory computer-readable medium of the present invention further stores instructions for producing the determined construction composition and/or admixture, the producing comprising

a) adding at least one construction admixture to the construction mixture to produce the construction composition, and causing the construction composition to be shipped to a job site, or

b) producing the determined construction admixture separately from the construction mixture, and causing the determined admixture to be shipped the job site, or

c) assembling the constituents of the construction admixture and causing the constituents to be shipped to the job site.

In another aspect, the present invention relates to a method for preparing a construction composition from a construction mixture and admixture, the method including:

- accessing at least one performance requirement of the construction composition,

- determining the construction composition including the construction mixture and the admixture, wherein the type and/or quantity of the admixture is suitable to be added to the construction mixture to meet the at least one performance requirement of the construction composition, said determination being implemented by a computer device including a predictive model, said determination including:

a) receiving at least one performance requirement of the construction composition, said at least one performance requirement being selected from processing requirements, exposure requirements, strength requirements, cost requirements, and/or environmental requirements; and

b) selecting, by the predictive model, an admixture type and/or quantity suitable to be added to the construction mixture, based on a comparison of the at least one performance requirement of the construction composition with reference performance requirements, said reference performance requirements including correlations between measured values of at least one property of a construction composition, composition of the construction mixture, and type and/or quantity of the admixture,

- assembling the determined construction composition by mixing the construction mixture and at least one admixture selected.

All features and embodiments described above also apply to this aspect.

Another aspect of the present invention is related to a system comprising a computer device whereby the system is configured for performing a method as described above.

Especially the system is a control unit of a concrete plant. Preferably, the system controls mixing machinery to produce the construction composition and/or construction mixture and/or admixture. This may be possible by transmitting instructions configured to cause the mixing machinery to acquire and mix raw materials specified in the output in amounts or ratios specified by the output.

In particular, the present invention relates to a system comprising a computer device, said system comprising

- means for carrying out a method to determine a construction composition or to determine a construction admixture,

- means for transferring at least one outputted construction composition and/or construction admixture to a mixing machinery,

- a mixing machinery, configured to produce an outputted construction composition and/or construction mixture and/or admixture by acquiring and mixing raw materials specified in the output.

All features and embodiments described above also apply to this aspect.

The construction composition and the respective environmental impact may be displayed and, if judged acceptable, the system may control mixing machinery to produce the construction composition, and/or construction mixture, and/or admixture.

Where a plurality of construction compositions is outputted, the system may allow one to be selected, in particular based on its environmental impact. However, a selection according to other parameters, for example the respective admixture, is also possible. Once a target construction composition is identified, the system may initiate a production process, which may involve generate instructions and/or controlling mixing machinery to produce the identified construction composition, and/or construction mixture, and/or admixture.

Acquiring raw materials and/or mixing raw materials may be controlled with suitable sensors.

Throughout the present context, a sensor is to be understood in a broad sense and stands for a device capable of determining a certain parameter. A sensor in particular is chosen from (i) a sensor capable of determining a chemical and/or physical property of at least one individual constituent of the construction mixture and/or admixture, and/or from (ii) a sensor capable of measuring environmental conditions during manufacture, transport, application and/or curing of the construction composition. For example, a sensor capable of determining a chemical and/or physical property of at least one individual constituent of the construction mixture is a particle size sensor, a particle shape sensor and/or a sensor for measuring an alkaline content. A sensor capable of measuring environmental conditions in particular is chosen from a sensor capable of determining a temperature, humidity, solar irradiance, air pressure, wind speed, wind direction, atmospheric composition, altitude, and/or other weather. Also, the sensor might be a traffic sensor, e.g. capable of measuring the traffic density and/or average speed of vehicles on a transport route.

The system may additionally comprise means to receive a report of a performance of the construction composition, and/or construction mixture, and/or admixture produced, and retrain the predictive model based on this performance.

**Brief description of the drawings**

The drawings used to explain the embodiments show:

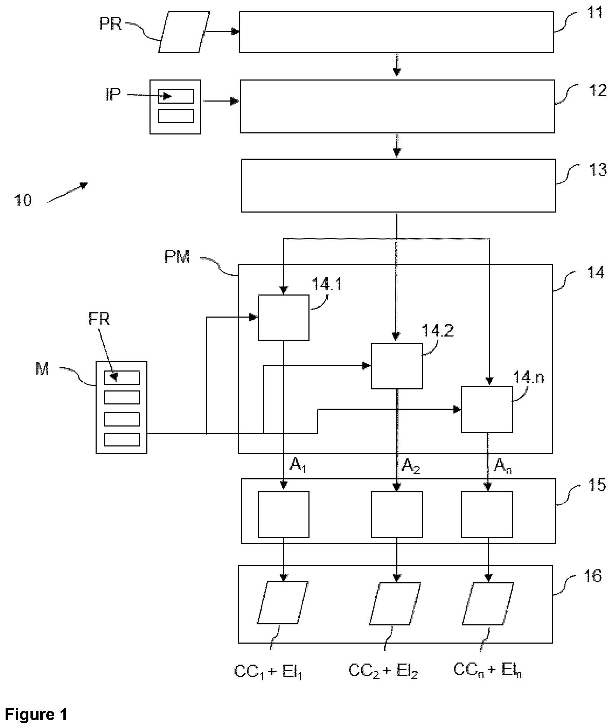

Fig. 1 a flow diagram of an inventive computer implemented method.

**Exemplary embodiments**

Fig. 1 shows a flow diagram of an inventive computer implemented method 10 to determine a construction composition.

In a first step (11), specifications comprising at least one performance requirement PR are received for the construction composition CC which comprises a construction mixture and an admixture. This is for example possible via a graphical user interface that is configured such that a predefined list of selectable performance requirements is presented to a user. Any inputs can be automatically transformed to numerical values during step 11.

In the second step 12, at least one input parameter **IP** related to the construction mixture is accessed. This is for example possible by accessing a database where input parameters are stored or via a graphical user interface.

In a third step 13, the specification for the construction composition **CC** and the at the at least one input parameter **IP** are provided to a predictive model **PM.**

In a fourth step 14, the predictive model **PM** determines a plurality of admixtures suitable to be added to the construction mixture to meet the performance requirement of the construction composition **CC.** Determination is done, for example, based on a set of functional relationships **FR**, which have been determined in advance by testing, and which are for example stored in a memory unit **M.** Functional relationships can, for example, be in the form of numerical equations, such as linear combinations.

For example, a functional relationship may be defined as follows:

$$c_{CEM\,I}A = c0 + a_i \cdot PR + b_i \cdot StR + c_i \cdot ExR + d_i \cdot EnR + e_i \cdot CR + f_i \cdot SeR$$

with $c_{CEM\,I}A$ = proportion of admixture A for a construction mixture comprising CEM I, whereby c0 = constant value; $a_i$, $b_i$, $c_i$, $d_i$, $e_i$, $f_i$ = coefficients of linear combination; PR = processing requirement, StR = strength requirement, ExR = exposure requirement, EnR = environmental requirement, CR 0 cost requirement, SeR = sensor reading (all represented by a numerical value).

However, other functional relationships may be used as well.

Specifically, in a first sub-step 14.1, a first admixture $A_1$ is determined, in a second sub-step 14.2, a second admixture $A_2$ is determined, and in an n-th step 14.n an n-th admixture $A_n$ is determined. Thereby, a plurality of admixtures is determined in step 14.

In a fifth step 15, the environmental impact of the cured construction composition **CC** comprising the construction mixture and the respective admixture is simulated. Thereby, n simulations are run, corresponding to the n admixtures determined, and a plurality of construction compositions **CC** with the respective environmental impact **EI** is generated.

In a sixth step 16, the plurality of construction compositions $CC_1$ to $CC_n$ with the respective environmental impact $EI_1$ to $EI_n$ is outputted, for example on a graphical user interface.

Thereafter, for example, a user can prepare a cementitious composition **CC** with a desired environmental impact **EI** by mixing the given construction mixture with a suitable admixture.

<u>Example 1</u>

The following specification for a concrete was received: exposure class XS1, slump class S3.

The following input parameters for the construction mixture were accessed: cement: 300 kg/m$^3$ of CEM 142.5 N, CEN standard sand (EN 196-1), basalt gravel (max. size 20 mm).

The following table 1 shows a plurality of admixtures determined and the simulated global warming potential.

*Table 1: results of admixture determination and simulation environmental impact of concrete*

| Admixture (type) | $CO_2$ equivalents per kg of admixture | Admixture dosage (w% rel. to cement) | $CO_2$ equivalents of concrete (kg/m$^3$) |
|---|---|---|---|
| Plasticizer: sulfonated naphthalin formaldehyde condensate (30% solids) | 0.51 | 0.80 | 1.23 |
| Superplasticizer: polycarboxylate ether 1 (30% solids) | 0.60 | 0.80 | 1.44 |

(continued)

| Admixture (type) | CO$_2$ equivalents per kg of admixture | Admixture dosage (w% rel. to cement) | CO$_2$ equivalents of concrete (kg/m$^3$) |
|---|---|---|---|
| Plasticizer: sulfonated naphthalin formaldehyde condensate (39% solids) | 0.49 | 0.50 | 0.74 |
| Superplasticizer: polycarboxylate ether 2 (14% solids) | 0.25 | 0.5 | 0.38 |

**Claims**

1. A computer implemented method to determine a construction composition which comprises a construction mixture and an admixture, said method comprising the steps of

    a) receiving a specification for the construction composition, said specification comprising at least one performance requirement for the construction composition,
    b) accessing at least one input parameter related to the construction mixture,
    c) providing the specification for the construction composition and at least one input parameter related to the construction mixture to a predictive model,
    d) determining, using the predictive model, a plurality of admixtures suitable to be added to the construction mixture to meet the performance requirement of the construction composition,
    e) simulating for each admixture an environmental impact of the cured construction composition comprising the construction mixture and the respective admixture,
    f) outputting the plurality of construction compositions and the respective environmental impact of the cured construction compositions.

2. The method as claimed in claim 1, **characterized in that** the method additionally comprises the step of

    g) deleting from the plurality of construction compositions at least one which has not the lowest environmental impact.

3. The method as claimed in any one of claims 1 or 2, **characterized in that** the method additionally comprises the steps of

    h) changing at least one input parameter related to the construction mixture in step b),
    i) repeating steps c) - f) and optionally g),
    j) comparing the lowest environmental impact of the cured construction compositions outputted in step f) and in step i),
    k) repeating steps h) - j) until the comparison in step j) shows no further reduction in environmental impact of the cured construction compositions,
    l) outputting the construction compositions and the respective environmental impact of the cured construction compositions obtained in step k).

4. The method as claimed in any of claims 1 - 3, **characterized in that** the environmental impact is selected from the global warming potential or the carbon footprint, in particular in CO$_2$ equivalents in kg per m$^3$.

5. The method as claimed in any of claims 1 - 4, **characterized in that** the at least one performance requirement for the construction composition is selected from at least one processing requirement, at least one strength requirement, at least one exposure requirement, at least one environmental requirement, at least one cost requirement, and/or at least one real-time sensor reading.

6. The method as claimed in any of claims 1 - 5, **characterized in that** the at least one input parameter related to the construction mixture is selected from a chemical and/or physical property of at least one individual constituent of the construction mixture.

7. The method as claimed in any of claims 1 - 6, **characterized in that** the at least one input parameter related to the construction mixture is accessed by measurement.

8. A non-transitory computer-readable medium storing instructions that, when executed by one or more processors, cause the one or more processors to

   i) receive a specification for a construction composition comprising a cementitious mixture and an admixture, said specification comprising at least one performance requirement for the construction composition,
   ii) access at least one input parameter related to the construction mixture,
   iii) provide the specification for the construction composition and at least one input parameter related to the construction mixture to a predictive model,
   iv) determine, using the predictive model, a plurality of admixtures suitable to be added to the construction mixture to meet the performance requirement of the construction composition,
   v) simulate for each admixture an environmental impact of the cured construction composition comprising the construction mixture and the respective admixture,
   vi) output the plurality of construction compositions and the respective environmental impact of the cured construction compositions.

9. A computer implemented method to determine a construction admixture, said method comprising the steps of

   1) receiving a specification for a construction composition comprising a construction mixture and the construction admixture, said specification comprising at least one performance requirement for the construction composition,
   2) accessing at least one input parameter related to the construction admixture,
   3) accessing at least one input parameter related to the construction mixture,
   4) providing the specification for the construction composition and the at least one input parameter related to the construction admixture and the construction mixture to a predictive model,
   5) determining, using the predictive model, a plurality of admixtures suitable to be added to the construction mixture to meet the performance requirement of the construction composition,
   6) simulating for each admixture an environmental impact of the cured construction composition comprising the construction mixture and the respective admixture,
   7) outputting the plurality of construction admixtures and the respective environmental impact of the cured construction compositions.

10. The method as claimed in claim 9, further comprising a step of
    8) selecting one or more characteristics in which the plurality of construction admixtures differs, the one or more characteristics not being an input parameter of the construction admixture and outputting a comparison of the plurality of construction admixtures based on the selected characteristics.

11. A non-transitory computer-readable medium storing instructions that, when executed by one or more processors, cause the one or more processors to

    i) receive a specification for a construction composition comprising a construction mixture and a construction admixture, said specification comprising at least one performance requirement for the construction composition,
    ii) access at least one input parameter related to the construction admixture,
    iii) access at least one input parameter related to the construction mixture,
    iv) provide the specification for the construction composition and the at least one input parameter related to the construction admixture and the construction mixture to a predictive model,
    v) determine, using the predictive model, a plurality of construction admixtures suitable to be added to the construction mixture to meet the performance requirement of the construction composition,
    vi) simulate for each construction admixture an environmental impact of the cured construction composition comprising the construction mixture and the respective construction admixture,
    vii) output the plurality of construction admixtures and the respective environmental impact of the cured construction compositions.

12. The non-transitory computer-readable medium according to claim 11, further storing instructions for producing the determined construction composition and/or construction admixture, the producing comprising

    a) adding at least one construction admixture to the construction mixture to produce the construction composition, and causing the construction composition to be shipped to a job site, or
    c) producing the determined construction admixture separately from the construction mixture, and causing the determined construction admixture to be shipped the job site, or

d) assembling the constituents of the construction admixture and causing the constituents to be shipped to the job site.

**13.** A system comprising a computer device, said system comprising

- means for carrying out a method to determine a construction composition as claimed in any of claims 1 - 7 and/or to determine a construction admixture as claimed in any of claims 9-10,
- means for transferring at least one outputted construction composition and/or construction admixture to a mixing machinery,
- a mixing machinery, configured to produce an outputted construction composition and/or construction mixture and/or admixture by acquiring and mixing raw materials specified in the output.

**14.** A method for preparing a construction composition from a construction mixture and admixture, the method including:

- accessing at least one performance requirement of the construction composition,
- determining the construction composition including the construction mixture and the admixture, wherein the type and/or quantity of the admixture is suitable to be added to the construction mixture to meet the at least one performance requirement of the construction composition, said determination being implemented by a computer device including a predictive model, said determination including:

a) receiving at least one performance requirement of the construction composition, said at least one performance requirement being selected from processing requirements, exposure requirements, strength requirements, cost requirements, and/or environmental requirements; and

b) selecting, by the predictive model, an admixture type and/or quantity suitable to be added to the construction mixture, based on a comparison of the at least one performance requirement of the construction composition with reference performance requirements, said reference performance requirements including correlations between measured values of at least one property of a construction composition, composition of the construction mixture, and type and/or quantity of the admixture,

- assembling the determined construction composition by mixing the construction mixture and at least one admixture selected.

**Figure 1**

Europäisches
Patentamt

European
Patent Office

Office européen
des brevets

**EUROPEAN SEARCH REPORT**

**Application Number**

EP 23 22 0106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG SHIQI ET AL: "Prediction and optimization model of sustainable concrete properties using machine learning, deep learning and swarm intelligence: A review", JOURNAL OF BUILDING ENGINEERING, vol. 80, 2 November 2023 (2023-11-02), page 108065, XP093170429, ISSN: 2352-7102, DOI: 10.1016/j.jobe.2023.108065 * abstract * * Sections 1-6 * | 1-14 | INV. C04B40/00 G06F30/20 G16C20/30 G16C60/00 G06F111/10 |
| X | M.A. DEROUSSEAU: "Computational design optimization of concrete mixtures: A review", CEMENT AND CONCRETE RESEARCH., vol. 109, 1 July 2018 (2018-07-01), pages 42-53, XP093168745, US ISSN: 0008-8846, DOI: 10.1016/j.cemconres.2018.04.007 * abstract * * Sections 1-5 * | 14 | |
| A | CN 116 882 199 A (CHINA WEST CONSTRUCTION GROUP BEIFANG CO LTD) 13 October 2023 (2023-10-13) * abstract * * paragraph [0038] - paragraph [0055]; figure 1 * | 1-14 | TECHNICAL FIELDS SEARCHED (IPC) C04B G16C G06F G06Q |
| A | KR 2023 0127540 A (KIM RAK HYUN [KR]; KUMOH NAT INST TECHNOLOGY IND ACAD COOP FOUND [KR]) 1 September 2023 (2023-09-01) * abstract * * paragraph [0026] - paragraph [0042] * | 1-14 | |

-/--

The present search report has been drawn up for all claims

| Place of search | Date of completion of the search | Examiner |
|---|---|---|
| Munich | 7 June 2024 | Bertolissi, Edy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2020/167728 A1 (CONSTRUCTION RESEARCH & TECHNOLOGY GMBH [DE]; DACZKO JOSEPH [US]) 20 August 2020 (2020-08-20) * abstract * * paragraph [0122] - paragraph [0137]; figure 6B * ----- | 1-14 | |
| A | CN 117 219 211 A (GUANGXI ROAD & BRIDGE ENG GROUP CO LTD) 12 December 2023 (2023-12-12) * abstract * * paragraph [0003] - paragraph [0064] * ----- | 1-14 | |
| | | | TECHNICAL FIELDS SEARCHED (IPC) |

The present search report has been drawn up for all claims

| Place of search | Date of completion of the search | Examiner |
|---|---|---|
| Munich | 7 June 2024 | Bertolissi, Edy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

# EP 4 574 801 A1

## ANNEX TO THE EUROPEAN SEARCH REPORT
## ON EUROPEAN PATENT APPLICATION NO.

EP 23 22 0106

07-06-2024

| Patent document cited in search report | | Publication date | Patent family member(s) | | Publication date |
|---|---|---|---|---|---|
| CN 116882199 | A | 13-10-2023 | NONE | | |
| KR 20230127540 | A | 01-09-2023 | NONE | | |
| WO 2020167728 | A1 | 20-08-2020 | AU | 2020222905 A1 | 26-08-2021 |
| | | | CA | 3129730 A1 | 20-08-2020 |
| | | | CN | 113614759 A | 05-11-2021 |
| | | | EP | 3924906 A1 | 22-12-2021 |
| | | | ES | 2964287 T3 | 05-04-2024 |
| | | | JP | 2022520077 A | 28-03-2022 |
| | | | US | 2022129797 A1 | 28-04-2022 |
| | | | WO | 2020167728 A1 | 20-08-2020 |
| CN 117219211 | A | 12-12-2023 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200402619 A1
- WO 2020167728 A1